# EUROPEAN PATENT APPLICATION

(11) **EP 1 967 183 A1**
(43) Date of publication of application: **10.09.2008**
(21) Application number: 06843647.6
(22) Date of filing: 28.12.2006
(51) Int. Cl.: A61K 9/26, A61K 9/14, A61K 31/4439, A61K 47/02, A61K 47/32, A61K 47/34, A61K 47/38, A61P 1/04, A61P 35/00

(54) **CONTROLLED RELEASE SOLID PREPARATION**

(30) Priority: 28.12.2005 JP 2005378242
(71) Applicant: Takeda Pharmaceutical Company Limited, Osaka-shi, Osaka 541-0045 (JP)
(72) Inventor: BANDO, Hiroto c/o TAKEDA PHARMACEUTICAL COMPANY LIMITED, Osaka-shi, Osaka 541-0045 (JP); KURASAWA, Takashi, Osaka-shi, Osaka 5328686 (JP)
(74) Representative: Wright, Robert Gordon McRae
(86) International application number: PCT/JP2006/326269
(87) International publication number: WO 2007/074909

(57) **Abstract**

The present invention provides a controlled release solid preparation superior in the stability of an active ingredient, which can exhibit pharmacological effects steadily and rapidly after administration, and shows a sustained pharmacological effect for a prolonged period of time: a controlled release solid preparation containing (1) an antacid, (2) an immediate-release part containing a compound unstable to acid and a basic substance, and (3) a sustained-release part containing a compound unstable to acid and a pH-independent material in combination.

## Description

### Technical Field

The present invention relates to a solid preparation. More particularly, the present invention relates to a controlled release solid preparation which is excellent in the stability of an active ingredient, can exhibit pharmacological effects steadily and rapidly after administration, and shows a sustained pharmacological effect for a prolonged period of time.

### Background Art

Since proton pump inhibitors (hereinafter sometimes referred to as PPI) such as benzimidazole compounds (e.g., lansoprazole, omeprazole, rabeprazole, pantoprazole, ilaprazole and the like) have a gastric acid secretion-inhibitory action, a gastric mucosa-protective action and the like, they have been widely used as therapeutic agents for peptic ulcer.
However, these compounds have poor stability, and are unstable to humidity, temperature, light, acid and the like. These compounds are particularly unstable to acid, and become extremely unstable as the pH of an aqueous solution or suspension thereof becomes low. When orally administered, therefore, these compounds may not be able to exhibit sufficient activity since they are decomposed by gastric acid and the like.
The stability of these compounds in preparations such as tablet, powder, fine granules, capsule and the like may become lower than that of the compounds themselves, due to strong interactions with other ingredient components in the preparation. As a result, color change and decomposition may be observed during production and preservation.
Various attempts have been made in the pharmaceutical preparations containing these compounds as active ingredients, so as to overcome the unstability of the compounds. For example, a tablet, granules or fine granules containing core particles containing PPI or a salt thereof or an optically active form thereof as an active ingredient, and a particular, pH-dependently soluble, release-controlling film (enteric film) have been disclosed (patent reference 1). Due to the enteric film, these preparations can suppress decomposition of the active ingredient by gastric acid and the like. However, since some time is required for dissolution of the enteric film in the gastrointestinal tract and absorption of the drug, rapid expression of the pharmacological effect in the early stage of the administration is hardly expected.
In the meantime, a gastrically-disintegrating solid preparation free of an enteric film, which contains an active ingredient unstable to acid and at least one kind of component selected from metal oxides and metal hydroxides is disclosed (patent reference 2). In addition, a chewable tablet free of an enteric film, which contains an active ingredient unstable to acid and at least one kind of component selected from alkaline earth metal carbonates, metal oxides and metal hydroxides is disclosed (patent reference 3). These preparations can suppress decomposition of the active ingredient by gastric acid and the like after administration, and are suitable for rapid expression of pharmacological effects upon administration. However, retention of the pharmacological effect for a prolonged period of time is difficult for these preparations.
Also, use of a basic inorganic salt for stabilization of the active ingredient in the preparation is disclosed (patent references 4 - 6, non-patent reference 1).
Furthermore, various preparations having plural different release control systems in combination are disclosed for the rapid expression of the pharmacological effect after administration and retention of the pharmacological effect for a prolonged period of time (patent references 7 - 11).
patent reference 1: JP-A-2004-292427
patent reference 2: JP-A-2003-327533
patent reference 3: JP-A-2005-154431
patent reference 4: JP-A-62-277322
patent reference 5: JP-A-2000-281564
patent reference 6: JP-A-2000-103731
patent reference 7: JP-A-2004-292442
patent reference 8: JP-A-2004-300149
patent reference 9: US Patent No. 6610323
patent reference 10: WO01/51050
patent reference 11: WO03/61584
non-patent reference 1: "DRUG DEVELOPMENT AND INDUSTRIAL PHARMACY", 18(13), 1437-1447 (1992)

### Disclosure of the Invention

### Problems to be Solved by the Invention

There is a demand for the development of a solid preparation comprising an active ingredient having high stability, wherein the active ingredient stably and rapidly expresses its pharmacological effect after administration, and the pharmacological effect is sustained for a prolonged period of time.
The present inventors have conducted intensive studies and found that a solid preparation comprising (1) an antacid, (2) an immediate-release part containing a compound unstable to acid and a basic substance, and (3) a sustained-release part containing a compound unstable to acid and a pH-independent material in combination shows high stability of the active ingredient, expresses a pharmacological effect of the active ingredient stably and rapidly after administration, and sustains the pharmacological effect for a prolonged period of time, which resulted in the completion of the present invention.

### Means of Solving the Problems

Accordingly, the present invention provides
[1] a controlled release solid preparation comprising (1) an antacid, (2) an immediate-release part containing a compound unstable to acid and a basic substance, and (3) a sustained-release part containing a compound unstable to acid and a pH-independent material in combination;
[2] the preparation of the above-mentioned [1], further comprising a basic substance in the sustained-release part;
[3] the preparation of the above-mentioned [1], wherein the pH-independent material is a hydrophilic polymer;
[4] the preparation of the above-mentioned [3], wherein the hydrophilic polymer is one kind or a mixture of two or more kinds selected from the group consisting of hydroxypropylcellulose, hydroxypropylmethylcellulose, methylcellulose, polyethylene oxide, sodium carboxymethylcellulose, ethyl acrylate-methyl methacrylate-trimethylammonioethyl methacrylate chloride copolymer, methyl methacrylate-ethyl acrylate copolymer and vinyl acetate-polyvinylpyrrolidone polymer matrix;
[5] the preparation of the above-mentioned [3], wherein the hydrophilic polymer is hydroxypropylmethylcellulose;
[6] the preparation of the above-mentioned [3], wherein the hydrophilic polymer is polyethylene oxide;
[7] the preparation of the above-mentioned [3], wherein the sustained-release part has a hydrophilic polymer content of about 5 wt% - about 95 wt%;
[8] the preparation of the above-mentioned [1], wherein the sustained-release part is a tablet, granule or fine granule having a pH-independent diffusion-controlling film;
[9] the preparation of the above-mentioned [8], wherein the pH-independent diffusion-controlling film contains one kind or a mixture of two or more kinds selected from the group consisting of an ethyl acrylate-methyl methacrylate-trimethylammonioethyl methacrylate chloride copolymer, a methyl methacrylate-ethyl acrylate copolymer and ethylcellulose;
[10] the preparation of the above-mentioned [1], wherein the compound unstable to acid is a proton pump inhibitor (PPI);
[11] the preparation of the above-mentioned [10], wherein the PPI is a compound represented by the formula (I):

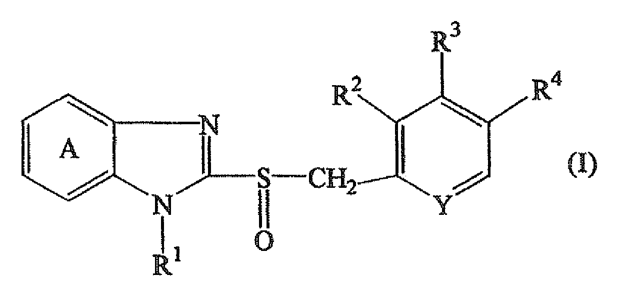

wherein ring A is a benzene ring optionally having substituent(s), R¹ is a hydrogen atom, an aralkyl group optionally having substituent(s), an acyl group or an acyloxy group, R², R³ and R⁴ are the same or different and each is a hydrogen atom, an alkyl group optionally having substituent(s), an alkoxy group optionally having substituent(s) or an amino group optionally having substituent(s), and Y is a nitrogen atom or CH, or an optically active form thereof or a salt thereof;
[12] the preparation of the above-mentioned [10], wherein the PPI is lansoprazole, omeprazole, rabeprazole, pantoprazole, ilaprazole or an optically active form thereof or a salt thereof;
[13] the preparation of the above-mentioned [1], wherein the antacid is at least one kind of component selected from the group consisting of a metal oxide, a metal hydroxide and an alkaline earth metal carbonate;
[14] the preparation of the above-mentioned [1], wherein a 1% aqueous solution or 1% aqueous suspension of the antacid has a pH of not less than 8.0;
[15] the preparation of the above-mentioned [13], wherein the metal oxide is at least one kind selected from the group consisting of magnesium oxide, magnesium silicate, dry aluminum hydroxide gel and magnesium aluminometasilicate;
[16] the preparation of the above-mentioned [13], wherein the metal hydroxide is at least one kind selected from the group consisting of magnesium hydroxide, aluminum hydroxide, synthetic hydrotalcite, coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate;
[17] the preparation of the above-mentioned [13], wherein the alkaline earth metal carbonate is calcium carbonate or magnesium carbonate;
[18] the preparation of the above-mentioned [1], wherein the content of the antacid is 5 mEq - 50 mEq;
[19] the preparation of the above-mentioned [1], wherein the weight ratio of the contents of the compound unstable to acid in the immediate-release part and the sustained-release part is 10:1 - 1:10;
[20] the preparation of the above-mentioned [1], which shows an increase in the intragastric average pH to 4 or above in 0.5 hr after oral administration to a mammal and a retention time at pH 4 or above of not less than 14 hr a day; and
[21] a solid preparation showing an increase in the intragastric average pH to 4 or above in 0.5 hr after oral administration to a mammal and a retention time at pH 4 or above of not less than 14 hr a day.

The present invention is explained in detail in the following.
The controlled release solid preparation of the present invention comprises (1) an antacid, (2) an immediate-release part containing a compound unstable to acid and a basic substance, and (3) a sustained-release part containing a compound unstable to acid and a pH-independent material in combination.

In the present specification, the terms "controlled release solid preparation of the present invention" and "solid preparation of the present invention" are used interchangeably unless otherwise specified.

### (1) Antacid

The solid preparation of the present invention contains an antacid. An antacid neutralizes the intragastric pH prior to the release of a compound unstable to acid, which is the active ingredient, in the stomach, whereby the residual ratio of the compound is increased and a stable and rapid pharmacological effect of the compound can be exhibited. As the antacid to be used in the present invention, at least one kind of component selected from the group consisting of metal oxide, metal hydroxide and alkaline earth metal carbonate is preferable.
As the above-mentioned metal oxide, at least one kind selected from the group consisting of magnesium oxide, magnesium silicate (2MgO·3SiO₂·xH₂O), dry aluminum hydroxide gel (Al₂O₃·xH₂O) and magnesium aluminometasilicate (Al₂O₃·MgO·2SiO₂· xH₂O), all for pharmaceutical agents, is preferably used.
Of these, magnesium oxide is more preferable. Magnesium oxide for medical use, which is superior in acid reactivity and has a neutralizing power, is preferable. As such magnesium oxide, one obtained by a general production method and a commercially available product can be used. What is called light burnt magnesia, which is obtained by calcination at a low temperature, is preferable. One obtained by calcination at a temperature of about 500°C - about 1000°C is generally preferable and, from the aspect of neutralizing power, one obtained by calcination at about 600°C - about 900°C is particularly preferable, and one obtained by calcination at about 800°C is most preferable. Of such magnesium oxides, one having a BET specific surface area of generally 10 - 50 m²/g, preferably 20 - 50 m²/g, is most preferable.
Here, the BET specific surface area is a specific surface area measured by a nitrogen gas adsorption method, where the specific surface area is measured based on the amount of nitrogen gas adsorbed by a certain amount of the surface of magnesium oxide and fine pores into which the nitrogen gas enters.
Examples of magnesium oxide include commercially available heavy magnesium oxide (manufactured by Kyowa chemical Industries Ltd.), heavy magnesium oxide (manufactured by Tomita Pharmaceutical Co., Ltd.), heavy magnesium N-oxide (manufactured by Kyowa chemical Industries Ltd.), light magnesium oxide (manufactured by Kyowa chemical Industries Ltd.) and the like. Particularly, heavy magnesium N-oxide (manufactured by Kyowa chemical Industries Ltd.) and the like are preferable.

As the metal hydroxide, at least one kind selected from the group consisting of magnesium hydroxide, aluminum hydroxide, synthetic hydrotalcite (Mg₆Al₂(OH)₁₆CO₃·4H₂O), coprecipitate of aluminum hydroxide and magnesium hydroxide, coprecipitate of aluminum hydroxide, magnesium carbonate and calcium carbonate, and coprecipitate of aluminum hydroxide and sodium hydrogen carbonate, all for pharmaceutical agents, is preferable. Of these, magnesium hydroxide is preferable in view of the disintegration property of preparation, dissolution property of compound unstable to acid and the like.

As the above-mentioned alkaline earth metal carbonate, calcium carbonate and magnesium carbonate for pharmaceutical grade, and the like can be used.

The above-mentioned metal oxide, metal hydroxide and alkaline earth metal carbonate may be used alone or two or more kinds thereof may be combined.
Some of the metal oxides and metal hydroxides polish surface of a formulating device during production to afford tablets having an entirely or partially dark surface, or a dark spot, line or plane, or attach to a punch for tabletting. These properties markedly reduce the producibility. Thus, it has been found that, when a metal oxide or metal hydroxide having abradability and punch-sticking property is to be selected, a metal oxide and a metal hydroxide free of such properties may be used in combination, or they may be subjected to wet or dry granulation together with an additive usable for pharmaceutical products (e.g., excipient, binder, disintegrant and the like explained in the below-mentioned (4)), whereby the polishing action and punch-sticking property can be suppressed.

The above-mentioned antacid preferably shows a pH of not less than 8.0, more preferably within the range of 8.0 - 12.0, when it is prepared into a 1% aqueous solution or 1% aqueous suspension.

The above-mentioned antacid is added, in an amount permitting rapid dissolution of the antacid to neutralize gastric acid, together with intragastric disintegration of solid preparation, and preferably prior to dissolution of the compound unstable to acid, so as to prevent unstabilization of the compound unstable to acid by exposure to the gastric acid. While the amount varies depending on the ability of each antacid to neutralize gastric acid, it is preferably 5 mEq - 50 mEq, more preferably 10 mEq - 50 mEq, in the solid preparation of the present invention.

### (2) Immediate-release part

The immediate-release part of the solid preparation of the present invention contains a compound unstable to acid and a basic substance.
In the immediate-release part, the release property of a compound unstable to acid, which is the active ingredient, is immediate-release. Here, the immediate-release means an elution ratio of the active ingredient at 30 min after the start of the test of not less than 85% when the Japanese Pharmacopoeia Dissolution Test Method 2 (Paddle Method) is performed using a suitable test solution (500 mL or 900 mL) under the conditions of paddle rotation of 100 rpm. As a test solution of a compound unstable to acid in an immediate-release part, for example, a test solution showing a concentration of the active ingredient of not more than 1/3 of the saturation solubility of the compound unstable to acid upon 100% dissolution thereof in the test solution is used. Preferably, 2nd fluid of the Japanese Pharmacopoeia Dissolution Test Method, or water is used.

### (2-1) Compound unstable to acid

The above-mentioned compound unstable to acid is not particularly limited, and may be any compound that becomes unstable when exposed to gastric acid. As such compound unstable to acid, for example, anti-inflammatory enzyme agents such as PPI, erythromycin antibacterial compounds, serrapeptase, semi-alkaline proteinase and the like, and the like can be used, PPI is preferable.
As PPI, for example, a compound represented by the following formula (I) [hereinafter sometimes to be referred to simply as compound (I)] is preferable.

Examples of compound (I) include a compound represented by the formula (I):

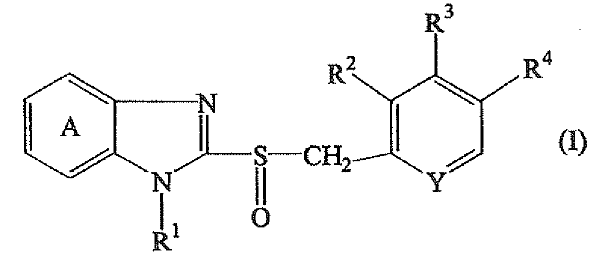

wherein ring A is a benzene ring optionally having substituent(s), R¹ is a hydrogen atom, an aralkyl group optionally having substituent(s), an acyl group or an acyloxy group, R², R³ and R⁴ are the same or different and each is a hydrogen atom, an alkyl group optionally having substituent(s), an alkoxy group optionally having substituent(s) or an amino group optionally having substituent(s), and Y is a nitrogen atom or CH, or an optically active form thereof or a salt thereof.

In the above-mentioned compound (I), examples of the "substituent" of the "benzene ring optionally having substituent(s)" for ring A include a halogen atom, a cyano group, a nitro group, an alkyl group optionally having substituent(s), a hydroxy group, an alkoxy group optionally having substituent(s), an aryl group, an aryloxy group, a carboxy group, an acyl group, an acyloxy group, a 5- to 10-membered heterocyclic group and the like. The benzene ring may be substituted by about 1 to 3 of these substituents. When the number of substituents is two or more, each substituent may be the same or different. Of these substituents, a halogen atom, an alkyl group optionally having substituent(s), an alkoxy group optionally having substituent(s) and the like are preferable.
Examples of the halogen atom include fluorine, chlorine, bromine atom and the like. Of these, a fluorine atom is preferable.
Examples of the "alkyl group" of the "alkyl group optionally having substituent(s)" include a C₁₋₇ alkyl group (e.g., methyl, ethyl, propyl, isopropyl, butyl, isobutyl, sec-butyl, tert-butyl, pentyl, hexyl, heptyl group etc.) and the like. Examples of the "substituent" of the "alkyl group optionally having substituent(s)" include a halogen atom, a hydroxy group, a C₁₋₆ alkoxy group (e.g., methoxy, ethoxy, propoxy, butoxy etc.), a C₁₋₆ alkoxy-carbonyl group (e.g., methoxycarbonyl, ethoxycarbonyl, propoxycarbonyl etc.), a carbamoyl group and the like, and the number of these substituents may be about 1 to 3. When the number of substituents is two or more, each substituent may be the same or different.
Examples of the "alkoxy group" of the "alkoxy group optionally having substituent(s)" include a C₁₋₆ alkoxy group (e.g., methoxy, ethoxy, propoxy, isopropoxy, butoxy, isobutoxy, pentoxy etc.) and the like. Examples of the "substituent" of the "alkoxy group optionally having substituent(s)" include those similar to the "substituent" of the above-mentioned "alkyl group optionally having substituent(s)", and the number of substituents is the same.
Examples of the "aryl group" include a C₆₋₁₄ aryl group (e.g., phenyl, 1-naphthyl, 2-naphthyl, biphenyl, 2-anthryl etc.) and the like.
Examples of the "aryloxy group" include a C₆₋₁₄ aryloxy group (e.g., phenyloxy, 1-naphthyloxy, 2-naphthyloxy etc.) and the like.
Examples of the "acyl group" include formyl, alkylcarbonyl, alkoxycarbonyl, carbamoyl, alkylcarbamoyl, alkylsulfinyl, alkylsulfonyl and the like.
Examples of the "alkylcarbonyl group" include a C₁₋₆ alkyl-carbonyl group (e.g., acetyl, propionyl etc.) and the like.
Examples of the "alkoxycarbonyl group" include a C₁₋₆ alkoxy-carbonyl group (e.g., methoxycarbonyl, ethoxycarbonyl, propoxycarbonyl, butoxycarbonyl etc.) and the like.
Examples of the "alkylcarbamoyl group" include an N-C₁₋₆ alkyl-carbamoyl group (e.g., methylcarbamoyl, ethylcarbamoyl group etc.), an N,N-di-C₁₋₆ alkyl-carbamoyl group (e.g., N,N-dimethylcarbamoyl, N,N-diethylcarbamoyl etc.) and the like.
Examples of the "alkylsulfinyl group" include a C₁₋₇ alkylsulfinyl group (e.g., methylsulfinyl, ethylsulfinyl, propylsulfinyl, isopropylsulfinyl etc.) and the like.
Examples of the "alkylsulfonyl group" include a C₁₋₇ alkylsulfonyl group (e.g., methylsulfonyl, ethylsulfonyl, propylsulfonyl, isopropylsulfonyl etc.) and the like.
Examples of the "acyloxy group" include alkylcarbonyloxy, alkoxycarbonyloxy, carbamoyloxy, alkylcarbamoyloxy, alkylsulfinyloxy, alkylsulfonyloxy and the like.
Examples of the "alkylcarbonyloxy group" include a C₁₋₆ alkyl-carbonyloxy group (e.g., acetyloxy, propionyloxy etc.) and the like.
Examples of the "alkoxycarbonyloxy group" include a C₁₋₆ alkoxy-carbonyloxy group (e.g., methoxycarbonyloxy, ethoxycarbonyloxy, propoxycarbonyloxy, butoxycarbonyloxy etc.) and the like.
Examples of the "alkylcarbamoyloxy group" include a C₁₋₆ alkyl-carbamoyloxy group (e.g., methylcarbamoyloxy, ethylcarbamoyloxy etc.) and the like.
Examples of the "alkylsulfinyloxy group" include a C₁₋₇ alkylsulfinyloxy group (e.g., methylsulfinyloxy, ethylsulfinyloxy, propylsulfinyloxy, isopropylsulfinyloxy etc.) and the like.
Examples of the "alkylsulfonyloxy group" include a C₁₋₇ alkylsulfonyloxy group (e.g., methylsulfonyloxy, ethylsulfonyloxy, propylsulfonyloxy, isopropylsulfonyloxy etc.) and the like.
Examples of the "5- to 10-membered heterocyclic group" include a 5- to 10-membered (preferably 5- or 6-membered) heterocyclic group containing, besides carbon atom, one or more (e.g., 1 - 3) hetero atoms selected from nitrogen atom, sulfur atom and oxygen atom and the like. Specific examples include 2- or 3-thienyl group, 2-, 3- or 4-pyridyl group, 2-or 3-furyl group, 1-, 2- or 3-pyrrolyl group, 2-, 3-, 4-, 5-or 8-quinolyl group, 1-, 3-, 4- or 5-isoquinolyl group, 1-, 2-or 3-indolyl group and the like. Of these, preferred is a 5-or 6-membered heterocyclic group such as 1-, 2- or 3-pyrrolyl group and the like.
Preferably, ring A is a benzene ring optionally having 1 or 2 substituents selected from a halogen atom, an optionally halogenated C₁₋₄ alkyl group, an optionally halogenated C₁₋₄ alkoxy group and a 5- or 6-membered heterocyclic group.

Examples of the "aralkyl group" of the "aralkyl group optionally having substituent(s)" for R¹ include a C₇₋₁₆ aralkyl group (e.g., C₆₋₁₀ aryl C₁₋₆ alkyl group such as benzyl, phenethyl etc., and the like) and the like. Examples of the "substituent" of the "aralkyl group optionally having substituent(s)" include substituents similar to the "substituent" of the above-mentioned "alkyl group optionally having substituent(s)", and the number of substituents is about 1 to 4. When the number of substituents is two or more, each substituent may be the same or different.
Examples of the "acyl group" for R¹ include the "acyl group" described as a substituent for the above-mentioned ring A and the like.
Examples of the "acyloxy group" for R¹ include the "acyloxy group" described as a substituent for the above-mentioned ring A and the like.
Preferable R¹ is a hydrogen atom.

Examples of the "alkyl group optionally having substituent(s)" for R², R³ or R⁴ include the "alkyl group optionally having substituent(s)" described as a substituent for the above-mentioned ring A and the like.
Examples of the "alkoxy group optionally having substituent (s)" for R², R³ or R⁴ include the "alkoxy group optionally having substituent(s)" described as the substituent for the above-mentioned ring A and the like.
Examples of the "amino group optionally having substituent(s)" for R², R³ or R⁴ include an amino group, a mono-C₁₋₆ alkylamino group (e.g., methylamino, ethylamino etc.), a mono-C₆₋₁₄ arylamino group (e.g., phenylamino, 1-naphthylamino, 2-naphthylamino etc.), a di-C₁₋₆ alkylamino group (e.g., dimethylamino, diethylamino etc.), a di-C₆₋₁₄ arylamino group (e.g., diphenylamino etc.) and the like.
Preferable R² is a C₁₋₆ alkyl group, a C₁₋₆ alkoxy group, a C₁₋₆ alkoxy-C₁₋₆ alkoxy group or a di-C₁₋₆ alkylamino group. More preferable R² is a C₁₋₃ alkyl group or a C₁₋₃ alkoxy group.
Preferable R³ is a hydrogen atom, a C₁₋₆ alkoxy-C₁₋₆ alkoxy group or an optionally halogenated C₁₋₆ alkoxy group. More preferable R³ is a C₁₋₃ alkoxy group which is optionally halogenated or substituted by a C₁₋₃ alkoxy group.
Preferable R⁴ is a hydrogen atom or C₁₋₆ alkyl group. More preferable R⁴ is a hydrogen atom or a C₁₋₃ alkyl group (particularly a hydrogen atom).
Preferable Y is a nitrogen atom.

Preferable compound of the formula (I) is a compound wherein ring A is a benzene ring optionally having substituent(s) selected from a halogen atom, an optionally halogenated C₁₋₄ alkyl group, an optionally halogenated C₁₋₄ alkoxy group and a 5- or 6-membered heterocyclic group, R¹ is a hydrogen atom, R² is a C₁₋₆ alkyl group, a C₁₋₆ alkoxy group, a C₁₋₆ alkoxy-C₁₋₆ alkoxy group or a di-C₁₋₆ alkylamino group, R³ is a hydrogen atom, a C₁₋₆ alkoxy-C₁₋₆ alkoxy group or an optionally halogenated C₁₋₆ alkoxy group, R⁴ is a hydrogen atom or a C₁₋₆ alkyl group, and Y is a nitrogen atom.

Of compound (I), a compound represented by the formula (Ia):

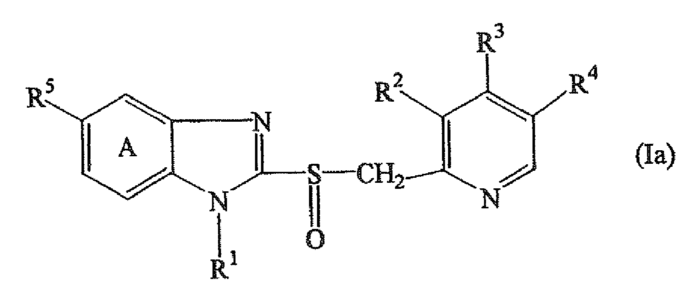

wherein R¹ is a hydrogen atom, R² is a C₁₋₃ alkyl group or a C₁₋₃ alkoxy group, R³ is a C₁₋₃ alkoxy group optionally halogenated or substituted by a C₁₋₃ alkoxy group, R⁴ is a hydrogen atom or a C₁₋₃ alkyl group, and R⁵ is a hydrogen atom, an optionally halogenated C₁₋₃ alkoxy group or a pyrrolyl group (e.g., 1-, 2-or 3-pyrrolyl group).
In the formula (Ia), a compound wherein R¹ is a hydrogen atom, R² is a C₁₋₃ alkyl group, R³ is an optionally halogenated C₁₋₃ alkoxy group, R⁴ is a hydrogen atom, and R⁵ is a hydrogen atom or an optionally halogenated C₁₋₃ alkoxy group is particularly preferable.

Specific examples of compound (I) include the following compounds.
2-[[[3-Methyl-4-(2,2,2-trifluoroethoxy)-2-pyridinyl]methyl]sulfinyl]-1H-benzimidazole, 2-[[(3,5-dimethyl-4-methoxy-2-pyridinyl)methyl]sulfinyl]-5-methoxy-1H-benzimidazole, 2-[[[4-(3-methoxypropoxy)-3-methyl-2-pyridinyl]methyl]sulfinyl]-1H-benzimidazole·sodium salt, 5-difluoromethoxy-2-[[(3,4-dimethoxy-2-pyridinyl)methyl]sulfinyl]-1H-benzimidazole and the like.
Of these compounds, 2-[[[3-methyl-4-(2,2,2-trifluoroethoxy)-2-pyridinyl]methyl]sulfinyl]-1H-benzimidazole(lansoprazole)is preferable.

Compound (I) may be a racemate or an optically active form such as R-form, S-form and the like. For example, compound (I) may be an optically active form such as (R)-2-[[[3-methyl-4-(2,2,2-trifluoroethoxy)-2-pyridinyl]methyl]sulfinyl]-1H-benzimidazole (to be sometimes referred to as lansoprazole R form) and the like. In addition, the optically active form is preferable.

As a salt of compound (I) or an optically active form thereof, a pharmaceutically acceptable salt is preferable. For example, salts of compound (I) or an optically active form thereof with an inorganic base, an organic base and a basic amino acid, and the like can be mentioned.
Preferable examples of the salt with inorganic base include alkali metal salts such as sodium salt, potassium salt and the like; alkaline earth metal salts such as calcium salt, magnesium salt and the like; ammonium salt and the like.
Preferable examples of the salt with organic base include salts with alkylamine (trimethylamine, triethylamine etc.), heterocyclic amine (pyridine, picoline etc.), alkanolamine (ethanolamine, diethanolamine, triethanolamine etc.), dicyclohexylamine, N,N'-dibenzylethylene diamine and the like.
Preferable examples of the salt with basic amino acid include salts with arginine, lysine, ornithine and the like.
Of these, preferred is an alkali metal salt or an alkaline earth metal salt. Particularly, a sodium salt is preferable.
Compound (I) can be produced by a method known per se, for example, the method described in JP-A-61-50978, US-B-4,628,098, JP-A-10-195068, WO98/21201 and the like or a method analogous thereto.
The optically active form of compound (I) can be obtained by a method such as an optical resolution method (fractional recrystallization, chiral column method, diastereomer method, a method using microorganism or enzyme etc.), asymmetric oxidation and the like. For example, a lansoprazole R form can be produced according to the methods described in WO00/78745, WO01/83473, WO01/87874 and WO02/44167.

The PPI to be used in the present invention is preferably selected from benzimidazole compounds having an antiulcer activity such as lansoprazole, omeprazole, rabeprazole, pantoprazole and ilaprazole, and optically active forms thereof and pharmaceutically acceptable salts thereof. More preferably, it is lansoprazole, omeprazole, rabeprazole or pantoprazole.

### (2-2) Basic substance

In the present invention, a basic substance is added to the immediate-release part to stabilize the above-mentioned compound unstable to acid in the preparation.
Examples of the above-mentioned basic substance include alkaline earth metal carbonates (e.g., calcium carbonate, magnesium carbonate for pharmaceutical agent etc.), tromethamol, disodium succinate, sodium hydrogenphosphate, trisodium phosphate, dipotassium phosphate, L-arginine and the like. Preferred is alkaline earth metal carbonate, and more preferred is calcium carbonate. These basic substances may be used alone or two or more kinds thereof may be used in combination.
The amount of the basic substance to be added is not particularly limited as long as it is sufficient to stabilize the above-mentioned substance unstable to acid. It is generally 1.0 wt% - 60 wt%, preferably 3.0 wt% - 50 wt%, relative to the total amount of the immediate-release part.
The above-mentioned basic substance should be distinguished from the antacid explained in the above-mentioned (1). For example, a substance used as the above-mentioned basic substance may also be used as the antacid of the above-mentioned (1) (e.g., the above-mentioned "alkaline earth metal carbonate"). When such substance is used as the antacid, it neutralizes the intragastric pH. On the other hand, when it is added to the immediate-release part as a basic substance, it stabilizes a compound unstable to acid in the preparation.

The form of the above-mentioned immediate-release part may be any. To achieve immediate release, granules, fine granules and the like are preferable.
The above-mentioned immediate-release part can be produced by a method known per se. For example, it can be produced by combining adequate amounts of a compound unstable to acid and a basic substance and, where necessary, an additive such as excipient, binder, disintegrant, lubricant, corrigent, colorant, flavor and the like, and granulating the mixture.
The above-mentioned granulation is preferably performed by a wet granulation method. The wet granulation method comprises dispersing or dissolving a mixture of the active ingredient and other components such as excipient and the like in water, a binder or a solvent, granulating the dispersion or solution, and drying same to give a granulation product such as granules, fine granules and the like. The wet granulation method can be performed according to a method known in the pharmaceutical field. As the granulation mechanism, for example, known methods such as extrusion, fluidizing, tumbling, centrifugation, stirring, spraying and the like can be used.

### (3) Sustained-release part

The sustained-release part in the solid preparation of the present invention contains a compound unstable to acid and a pH-independent material.
In the sustained-release part, the release property of a compound unstable to acid, which is the active ingredient, is sustained-release. The sustained-release means an elution ratio of the active ingredient at 30 min after the start of the test of less than 85% when the Japanese Pharmacopoeia Dissolution Test Method 2 (Paddle Method) is performed using a suitable test solution (500 mL or 900 mL) under the conditions of paddle rotation of 100 rpm. As the test solution here, those similar to the ones recited in the explanation of the above-mentioned (2) Immediate-release part can be used.

### (3-1) Compound unstable to acid

As the above-mentioned compound unstable to acid, those similar to the compounds unstable to acid recited in the explanation of the above-mentioned (2-1) can be used, with preference given to PPI. The compound unstable to acid contained in the sustained-release part may be the same as or different from the compound unstable to acid contained in the immediate-release part.

### (3-2) pH-independent material

The pH-independent material to be used for the sustained-release part of the solid preparation of the present invention is a substrate capable of releasing an active ingredient in a sustained manner without showing varying release property of the active ingredient even when, for example, the pH of the external environment changes due to the movement in the gastrointestinal tract and the like. Examples of such pH-independent material include a mixture of one or more kinds selected from a hydrophilic polymer, a hydrophobic polymer and an amphiphilic polymer, and particularly, a hydrophilic polymer is more preferable.

The above-mentioned hydrophilic polymer is a polymer that becomes a hydrogel upon water absorption and can control release of the active ingredient in the sustained-release part, or a polymer that is dissolve in water and can control release of the active ingredient in the sustained-release part.
The above-mentioned hydrophobic polymer means a polymer insoluble in water but soluble in an organic solvent miscible with water, and capable of controlling release of the active ingredient in the sustained-release part.
The above-mentioned amphiphilic polymer has both a hydrophilic group and a hydrophobic group, and can control release of the active ingredient in the sustained-release part.
In the solid preparation of the present invention, the release rate of the compound unstable to acid from the sustained-release part can be adjusted to any level by controlling the viscosity and addition amount of a hydrophilic polymer, a hydrophobic polymer and an amphiphilic polymer.
The viscosity of the above-mentioned hydrophilic polymer, for example, as a viscosity of a 2 wt% aqueous solution (measurement temperature: 20°C) is preferably not less than 1 mPa·s, more preferably not less than 4 mPa·s.
In addition, the content of the above-mentioned hydrophilic polymer in the sustained-release part is generally about 5 wt% - about 95 wt%, preferably about 10 wt% - about 50 wt%, more preferably about 20 wt% - about 40 wt%.

The above-mentioned hydrophilic polymer is preferably a mixture of one or more kinds selected from the group consisting of hydroxypropylcellulose, hydroxypropylmethylcellulose, methylcellulose, polyethylene oxide, sodium carboxymethylcellulose, ethyl acrylate-methyl methacrylate-trimethylammonioethyl methacrylate chloride copolymer, methyl methacrylate-ethyl acrylate copolymer and vinyl acetate-polyvinylpyrrolidone polymer matrix.

Examples of the above-mentioned hydroxypropylcellulose include HPC-SSL (trade name, manufactured by Nippon Soda Co., Ltd.) (viscosity of 2 wt% aqueous solution at 20°C: 2.0 - 2.9 mPa·s), HPC-SL (trade name, manufactured by Nippon Soda Co., Ltd.) (viscosity of 2 wt% aqueous solution at 20°C: 3.0 - 5.9 mPa·s), HPC-L (trade name, manufactured by Nippon Soda Co., Ltd.) (viscosity of 2 wt% aqueous solution at 20°C: 6.0 - 10.0 mPa·s), HPC-M (trade name, manufactured by Nippon Soda Co., Ltd.) (viscosity of 2 wt% aqueous solution at 20°C: 150 - 400 mPa·s), HPC-H (trade name, manufactured by Nippon Soda Co., Ltd.) (viscosity of 2 wt% aqueous solution at 20°C: 1000 - 4000 mPa·s) and the like.

Examples of the above-mentioned hydroxypropylmethylcellulose include TC-5S (trade name, manufactured by Shin-Etsu Chemical Co., Ltd.) (viscosity of 2 wt% aqueous solution at 20°C: about 15 mPa·s), TC-5R (trade name, manufactured by Shin-Etsu Chemical Co., Ltd.) (viscosity of 2 wt% aqueous solution at 20°C: about 6 mPa·s), TC-5E (trade name, manufactured by Shin-Etsu Chemical Co., Ltd.) (viscosity of 2 wt% aqueous solution at 20°C: about 3 mPa·s), TC-5MW (trade name, manufactured by Shin-Etsu Chemical Co., Ltd.) (viscosity of 2 wt% aqueous solution at 20°C: about 4 mPa·s), Metolose 60SH-50 (trade name, manufactured by Shin-Etsu Chemical Co., Ltd.) (viscosity of 2 wt% aqueous solution at 20°C: about 50 mPa·s), Metolose 65SH-50 (trade name, manufactured by Shin-Etsu Chemical Co., Ltd.) (viscosity of 2 wt% aqueous solution at 20°C: about 50 mPa·s), Metolose 90SH-100 (trade name, manufactured by Shin-Etsu Chemical Co., Ltd.) (viscosity of 2 wt% aqueous solution at 20°C: about 100 mPa·s), Metolose 65SH-400 (trade name, manufactured by Shin-Etsu Chemical Co., Ltd.) (viscosity of 2 wt% aqueous solution at 20°C: about 400 mPa·s), Metolose 90SH-400 (trade name, manufactured by Shin-Etsu Chemical Co., Ltd.) (viscosity of 2 wt% aqueous solution at 20°C: about 400 mPa·s), Metolose 65SH-1500 (trade name, manufactured by Shin-Etsu Chemical Co., Ltd.) (viscosity of 2 wt% aqueous solution at 20°C: about 1500 mPa·s), Metolose 60SH-4000 (trade name, manufactured by Shin-Etsu Chemical Co., Ltd.) (viscosity of 2 wt% aqueous solution at 20°C: about 4000 mPa·s), Metolose 65SH-4000 (trade name, manufactured by Shin-Etsu Chemical Co., Ltd.) (viscosity of 2 wt% aqueous solution at 20°C: about 4000 mPa·s), Metolose 90SH-4000 (trade name, manufactured by Shin-Etsu Chemical Co., Ltd.) (viscosity of 2 wt% aqueous solution at 20°C: about 4000 mPa·s), Metolose 90SH-30000 (trade name, manufactured by Shin-Etsu Chemical Co., Ltd.) (viscosity of 2 wt% aqueous solution at 20°C: about 30000 mPa·s) and the like.

Examples of the above-mentioned methylcellulose include Metolose SM15 (trade name, manufactured by Shin-Etsu Chemical Co., Ltd.) (viscosity: about 15m Pa·s, 2 wt% aqueous solution, 20°C), Metolose SM25 (trade name, manufactured by Shin-Etsu Chemical Co., Ltd.) (viscosity of 2 wt% aqueous solution at 20°C: about 25 mPa·s), Metolose SM100 (trade name, manufactured by Shin-Etsu Chemical Co., Ltd.) (viscosity of 2 wt% aqueous solution at 20°C: about 100 mPa·s), Metolose SM400 (trade name, manufactured by Shin-Etsu Chemical Co., Ltd.) (viscosity of 2 wt% aqueous solution at 20°C: about 400 mPa·s), Metolose SM1500 (trade name, manufactured by Shin-Etsu Chemical Co., Ltd.) (viscosity of 2 wt% aqueous solution at 20°C: about 1500 mPa·s), Metolose SM4000 (trade name, manufactured by Shin-Etsu Chemical Co., Ltd.) (viscosity of 2 wt% aqueous solution at 20°C: about 4000 mPa·s), Metolose SM8000 (trade name, manufactured by Shin-Etsu Chemical Co., Ltd.) (viscosity of 2 wt% aqueous solution at 20°C: about 8000 mPa·s) and the like.

Examples of the above-mentioned polyethylene oxide include POLYOX WSR N-12K (trade name, manufactured by Union Carbide Corporation) (viscosity of 2 wt% aqueous solution at 20°C: 400 - 800 mPa·s), POLYOX WSR N-60K (trade name, manufactured by Union Carbide Corporation) (viscosity of 2 wt% aqueous solution at 20°C: 2000 - 4000 mPa·s), POLYOX WSR 301 (trade name, manufactured by Union Carbide Corporation) (viscosity of 1 wt% aqueous solution at 25°C: 1500 - 4500 mPa· s), POLYOX WSR Coagulant (trade name, manufactured by Union Carbide Corporation) (viscosity of 1 wt% aqueous solution at 25°C: 4500 - 7500 mPa·s), POLYOX WSR 303 (trade name, manufactured by Union Carbide Corporation) (viscosity of 1 wt% aqueous solution at 25°C: 7500 - 10000 mPa·s), POLYOX WSR 308 (trade name, manufactured by Union Carbide Corporation) (viscosity of 1 wt% aqueous solution at 25°C: 10000 - 15000 mPa·s) and the like.

Examples of the above-mentioned sodium carboxymethylcellulose include Sunrose F-150MC (trade name, manufactured by Nippon Paper) (viscosity of 1 wt% aqueous solution at 25°C: 1200 - 1800 mPa·s), Sunrose F-300MC (trade name, manufactured by Nippon Paper) (viscosity of 1 wt% aqueous solution at 25°C: 2500 - 3000 mPa·s), Sunrose F-1000MC (trade name, manufactured by Nippon Paper) (viscosity of 1 wt% aqueous solution at 25°C: 8000 - 12000 mPa·s) and the like.

Examples of the above-mentioned ethyl acrylate-methyl methacrylate-trimethylammonioethyl methacrylate chloride copolymer include Eudragit RLPO (trade name, manufactured by Rohm), Eudragit RL100 (trade name, manufactured by Rohm), Eudragit RL30D (trade name, manufactured by Rohm), Eudragit RSPO (trade name, manufactured by Rohm), Eudragit RS100 (trade name, manufactured by Rohm), Eudragit RS30D (trade name, manufactured by Rohm) and the like.

Examples of the above-mentioned methyl methacrylate-ethyl acrylate copolymer include Eudragit NE30D (trade name, manufactured by Rohm) and the like.

Examples of the above-mentioned vinyl acetate-polyvinylpyrrolidone polymer matrix include Kollidon VA64 (trade name, manufactured by BASF Takeda vitamin) and the like.

These hydrophilic polymers may be used alone or in a mixture of two or more kinds thereof at an appropriate ratio.

As the above-mentioned hydrophilic polymer, hydroxypropylmethylcellulose or polyethylene oxide is more preferable.

The above-mentioned hydrophobic polymer is preferably a mixture of one or more kinds selected from the group consisting of ethylcellulose, cellulose acetate and polyvinyl acetate.
Examples of the above-mentioned ethylcellulose include Ethocel 4P (trade name, manufactured by Nissin Kasei Kogyo Co., Ltd.) (viscosity of 5 wt% (80% toluene/20% alcohol) solution at 25°C: about 3-5.5 cP), Ethocel 7P (trade name, manufactured by Nissin Kasei Kogyo Co., Ltd.) (viscosity of 5 wt% (80% toluene/20% alcohol) solution at 25°C: about 6-8 cP), Ethocel 10P (trade name, manufactured by Nissin Kasei Kogyo Co., Ltd.) (viscosity of 5 wt% (80% toluene/20% alcohol) solution at 25°C: about 9-11 cP), Ethocel 20P (trade name, manufactured by Nissin Kasei Kogyo Co., Ltd.) (viscosity of 5 wt% (80% toluene/20% alcohol) solution at 25°C: about 18-22 cP), Ethocel 45P (trade name, manufactured by Nissin Kasei Kogyo Co., Ltd.) (viscosity of 5 wt% (80% toluene/20% alcohol) solution at 25°C: about 41-49 cP), Ethocel 100P (trade name, manufactured by Nissin Kasei Kogyo Co., Ltd.) (viscosity of 5 wt% (80% toluene/20% alcohol) solution at 25°C: about 90-110 cP) and the like.
Examples of the above-mentioned cellulose acetate include cellulose acetate CA-398-3 (trade name, manufactured by Eastman) and the like.
Examples of the above-mentioned polyvinyl acetate include Kollicoat SR30D (trade name, manufactured by BASF) and the like.
These hydrophobic polymers may be used alone or in a mixture of two or more kinds thereof at an appropriate ratio.
As the above-mentioned hydrophobic polymer, ethylcellulose or polyvinyl acetate is more preferable.

The above-mentioned amphiphilic polymer is preferably a mixture of one or more kinds selected from polyoxyethylene polyoxypropylene glycol copolymers.
Examples of the above-mentioned polyoxyethylene polyoxypropylene glycol copolymer include pluronic F-68 (trade name, manufactured by BASF), pluronic F-127 (trade name, manufactured by BASF) and the like. These amphiphilic polymers may be used alone or in a mixture of two or more kinds thereof at an appropriate ratio.
As the above-mentioned amphiphilic polymer, pluronic F-127 is more preferable.

The method of forming the above-mentioned sustained-release part is not particularly limited, and the part can be formed by a method generally used in the pharmaceutical field. For example, a compound unstable to acid and a pH-independent material and, where necessary, the below-mentioned basic substance, various additives and the like are mixed by a general method in the pharmaceutical field, and the mixture is tabletted, granulated or finely granulated by a general method in the pharmaceutical field, for example, the method described in the Japanese Pharmacopoeia 14th Revision, Preparation General Principles, whereby a sustained-release part in the form of tablet, granules or fine granules can be obtained.
Particularly, to afford a sustained-release part in the form of granules or fine granules, granulation by a wet granulation method is preferable. The wet granulation method comprises dispersing or dissolving a mixture containing the active ingredient and other components (excipient and the like) in water, a binder or a solvent, granulating the dispersion or solution, and drying same to give a granulation product such as granules, fine granules and the like. The wet granulation method can be performed according to a method known in the pharmaceutical field. As the granulation mechanism, for example, known methods such as extrusion, fluidizing, tumbling, centrifugation, stirring, spraying and the like can be used.

The above-mentioned sustained-release part is preferably a tablet, granules or fine granules having a pH-independent diffusion-controlling film.
Here, the "diffusion-controlling film" to be used in the present specification generally means a film which is undissolved by itself and controls release of the active ingredient by diffusion through the film itself or fine pores produced in the film.
The above-mentioned pH-independent diffusion-controlling film is not particularly limited as long as it is a diffusion-controlling film constituted from the above-mentioned pH-independent material and stably can control release of a compound unstable to acid without depending on pH. As the pH-independent diffusion-controlling film, for example, a film containing a mixture of one or more kinds selected from the group consisting of an ethyl acrylate-methyl methacrylate-trimethylammonioethyl methacrylate chloride copolymer and a methyl methacrylate-ethyl acrylate copolymer explained as the above-mentioned hydrophilic polymer, and ethylcellulose as a hydrophobic polymer is preferable.

Since a compound unstable to acid is stabilized in the preparation in the present invention, a basic substance may be added to a sustained-release part as necessary. As the above-mentioned basic substance, those similar to the basic substances explained in the above-mentioned (2-2) can be mentioned, and magnesium carbonate is preferable. The basic substance to be contained in the sustained-release part may be the same as or different from the basic substance to be contained in the immediate-release part.

The form of such sustained-release part is, for example, a form comprising a pH-independent diffusion-controlling film on the surface of a core particle containing a compound unstable to acid and a basic substance as necessary.
Examples of such core particle include tablet, granules or fine granules comprising an inactive carrier [e.g., Nonpareil (Nonpareil-101 (particle size 850-710, 710-500, 500-355), Nonpareil-103 (particle size 850-710, 710-500, 500-355), Nonpareil-105 (particle size 300-180), manufactured by Freund Industry Co., Ltd.), Celphere (CP-507 (particle size 500-710), CP-305 (particle size 300-500), CP-203 (particle size 150-300), CP-102 (particle size 106-212), SCP-100 (particle size 75-212), manufactured by Asahi Kasei Chemicals Co., Ltd.) etc.] as a core, and a coating solution containing a compound unstable to acid and a basic substance as necessary, which is applied to the surface of the core; a tablet prepared using the granules or fine granules; particles obtained by granulating the active ingredient and an excipient generally used for preparation making and the like.
The core particle can be produced, for example, by the method described in JP-A-63-301816. For example, when a core particle is to be obtained by applying the above-mentioned coating solution on the core of the inactive carrier, a core particle containing a compound unstable to acid and a basic substance as necessary can be prepared by wet granulation using a rotating fluidized bed coater (SPIR-A-FLOW, manufactured by Freund Industry Co., Ltd.), a centrifugal fluid bed granulator (CF-mini, CF-360, manufactured by Freund Industry Co., Ltd.), a rotating fluidized bed granulator (POWREX MP-10) and the like. Alternatively, the above-mentioned coating solution may be sprayed to form a coating while spraying a solution containing a binder and the like on the core of the inactive carrier. In this case, the production apparatus is not limited. However, a centrifugal fluid bed granulator and the like is preferably used. Alternatively, coating by the above-mentioned two kinds of apparatuses may be combined and the compound unstable to acid and a basic substance where necessary may be applied in two steps.
Alternatively, the core particle may be prepared by dry granulation using a roller compactor and the like.
On the other hand, when an inactive carrier core is not used, a core particle containing a compound unstable to acid can be obtained by adding a compound unstable to acid and, where necessary, a basic substance and an excipient such as lactose, sucrose, mannitol, cornstarch, crystalline cellulose and the like to a binder such as hydroxypropylmethylcellulose, hydroxypropylcellulose, methylcellulose, polyvinyl alcohol, macrogol, pluronic F68, gum arabic, gelatin, starch and the like and, where necessary, a disintegrant such as sodium carboxymethylcellulose, calcium carboxymethylcellulose, sodium croscarboxymethylcellulose (Ac-Di-Sol, manufactured by FMC International), polyvinylpyrrolidone, low-substituted hydroxypropylcellulose and the like, and granulating the mixture by a stirring granulator, wet extrusion-granulator, fluidized bed granulator and the like.
The obtained core particle is sieved as necessary to give a particle having a desired particle size. While the particle size is not particularly limited, it is generally about 50 µm - about 5 mm, preferably about 100 µm - about 3 mm, more preferably about 100 µm - about 2 mm.
Subsequently, the surface of the above-mentioned core particle is coated with a coating solution containing a pH-independent material by a method known in the pharmaceutical field to give a sustained-release part having a pH-independent diffusion-controlling film on the surface of a core particle containing a compound unstable to acid.

The above-mentioned "Having" film includes having not only a film-like coating but also a coating with a higher thickness, and further, not only a cover film on the entire surface of a core particle containing a compound unstable to acid and a basic substance but also a cover film on a part of the surface of the core particle (e.g., cover film on most of the surface (not less than 80%) of the core particle, though partially uncoated).

In the above-mentioned sustained-release part, an intermediate layer may be formed as necessary between a core particle containing a compound unstable to acid and a pH-independent diffusion-controlling film. By shutting off a direct contact of a core particle with a film by an intermediate layer, the stability of a compound unstable to acid, which is the active ingredient, can be improved.
Examples of the material for the above-mentioned intermediate layer include a blend of a polymer substrate such as low-substituted hydroxypropylcellulose, hydroxypropylcellulose, hydroxypropylmethylcellulose (e.g., TC-5 and the like), polyvinylpyrrolidone, polyvinyl alcohol, methylcellulose, hydroxyethylmethylcellulose and the like, and saccharides such as sucrose [purified sucrose (pulverized (powder sugar) or unpulverized) and the like], starch sugar such as cornstarch and the like, lactose, honey and sugar alcohol (D-mannitol, erythritol etc.) and the like at an appropriate ratio and the like. Where necessary, moreover, the intermediate layer may contain an excipient (e.g., masking agent (titanium oxide etc.), an antistat (titanium oxide, talc etc.)) and the like as appropriate.
The amount of coating of the intermediate layer is generally about 0.02 part by weight - about 1.5 parts by weight, preferably about 0.05 - about 1 part by weight, relative to 1 part by weight of the core particle. The intermediate layer can be applied by a conventional method. For example, the components of the intermediate layer are preferably diluted with purified water and the like, and sprayed as a liquid. In this case, it is more preferable to apply the liquid while spraying a binder such as hydroxypropylcellulose and the like during coating.
The intermediate layer may consist of a single layer or plural layers.

### (4) Additive

In the solid preparation of the present invention, examples of the additive that may be contained in the immediate-release part and/or sustained-release part as necessary include excipients (e.g., glucose, fructose, lactose, saccharose, D-mannitol, erythritol, maltitol, trehalose, sorbitol, cornstarch, potato starch, wheat starch, rice starch, microcrystalline cellulose, silicic anhydride, anhydrous calcium phosphate, precipitated calcium carbonate, calcium silicate, amorphous colloidal silica dioxide (e.g., aerosil etc.) and the like); binders (e.g., polyvinylpyrrolidone, polyvinyl alcohol, partly pregelatinized starch, pregelatinized starch, sodium alginate, pullulan, gum arabic powder, gelatin etc.); disintegrants (e.g., low-substituted hydroxypropylcellulose, carmellose, carmellose calcium, sodium carboxymethyl starch, croscarmellose sodium, crospovidone, hydroxypropyl starch etc.); corrigents (e.g., citric acid, ascorbic acid, tartaric acid, malic acid, aspartame, acesulfame potassium, thaumatin, sodium saccharin, dipotassium glycyrrhizinate, sodium glutamate, sodium 5'-inosinate, sodium 5'-guanylate etc.); surfactants (e.g., polysorbate, polyoxyethylene-polyoxypropylene copolymer, sodium lauryl sulfate etc.); flavors (e.g., lemon oil, orange oil, menthol, peppermint etc.); lubricants (e.g., magnesium stearate, sucrose esters of fatty acids, sodium stearyl fumarate, stearic acid, talc, polyethylene glycol etc.); colorants (e.g., Food Color Yellow No. 5, Food Color Blue No. 2, diiron trioxide, yellow ferric oxide etc.); antioxidants (e.g., sodium ascorbate, L-cysteine, sodium sulfite etc.) and the like. While the particle size of these additives is not particularly limited, it is preferably not more than 500 µm in view of the particle producibility and easy administration.

### (5) Production method of solid preparation

The solid preparation of the present invention can be obtained by combining the above-mentioned antacid, immediate-release part and sustained-release part.
The solid preparation of the present invention can be produced by any method known in the pharmaceutical field. In addition, the combination of the above-mentioned antacid, immediate-release part and sustained-release part may be any and, for example, the following combinations can be mentioned.
(i) When the sustained-release part is a tablet, the solid preparation of the present invention (tablet) containing a sustained-release part as an inner-core matrix and an antacid and an immediate-release part as an outer layer can be obtained by filling a mixture of an antacid and a granulated powder for immediate-release part (granules or fine granules) in a die, placing a tablet for a sustained-release part thereon, filling a mixture of an antacid and a granulated powder for immediate-release part (granules or fine granules) thereon, and punching them by a method known in the pharmaceutical field.
(ii) When the sustained-release part is a tablet, the solid preparation of the present invention (tablet) in the form of a two-layer tablet having a layer containing an antacid and a sustained-release part and an immediate-release part layer can be obtained by filling an antacid and a granulated powder for an immediate-release part (granules or fine granules) in a die, placing a tablet to be a sustained-release part thereon and punching them by a method known in the pharmaceutical field. The tablet is not limited to a two-layer tablet and may be a multi-layer tablet formed by adding, where necessary, a layer containing an antacid and a sustained-release part and an immediate-release part layer. A multi-layer tablet can also be punched by a method similar to that used for the two-layer tablet.
(iii) When the sustained-release part is granules or fine granules, the solid preparation of the present invention (granules or fine granules), wherein an antacid, an immediate-release part and a sustained-release part are uniformly dispersed, can be obtained by mixing the antacid, the granulated powder for sustained-release part (granules or fine granules) and the granulated powder for immediate-release part (granules or fine granules) by a method known in the pharmaceutical field.
(iv) The solid preparation of the present invention (tablet), wherein the antacid, immediate-release part and sustained-release part are uniformly dispersed in the tablet, can also be obtained by further punching the solid preparation (granules or fine granules) obtained in the above-mentioned (iii).
(v) The solid preparation of the present invention (capsule) can also be obtained by filling the solid preparation (granules or fine granules) obtained in the above-mentioned (iii) in a capsule.
Particularly, the solid preparation of the present invention (tablet) obtained in the above-mentioned (i) is preferable.

While the total amount of the compound unstable to acid in the solid preparation of the present invention varies depending on the kind, dose and the like of the compound unstable to acid, it is generally 1.0 wt% - 60 wt%, preferably 10 wt% - 40 wt%, of the total amount of the solid preparation of the present invention.
In the solid preparation of the present invention, moreover, the content weight ratio of the compound unstable to acid in the immediate-release part and the sustained-release part is preferably 10:1 - 1:10, more preferably 5:1 - 1:5, most preferably 2:1 - 1:5.

### (6) Applicable disease, administration mode, dose and the like of the solid preparation of the present invention

When the solid preparation of the present invention contains a PPI such as a compound represented by the formula (I) as a compound unstable to acid, the compound is superior in the antiulcer activity, gastric acid secretion-inhibitory action, mucosa-protecting action, anti-Helicobacter pylori activity and the like, and shows low toxicity. Hence, the solid preparation of the present invention is useful as a pharmaceutical agent. In this case, the solid preparation of the present invention can be orally administered to a mammal (e.g., human, monkey, sheep, horse, dog, cat, rabbit, rat, mouse and the like) for the treatment or prophylaxis of peptic ulcer (e.g., gastric ulcer, duodenal ulcer, anastomotic ulcer, Zollinger-Ellison syndrome and the like), gastritis, GERD (Gastroesophageal Reflux Diseases; erosive esophagitis, esophageal reflux unaccompanied by esophagitis (Symptomatic GERD) and the like), NUD (Non Ulcer Dyspepsia), gastric cancer (including gastric cancer associated with promotion of interleukin-1β production by genetic polymorphism of interleukin-1), stomach MALT lymphoma and the like, eradication or as an aid for eradication of Helicobacter pylori, suppression of peptic ulcer, acute stress ulcer and hemorrhagic stomach, upper gastrointestinal hemorrhage due to invasive stress (stress caused by major surgery requiring postoperative intensive management or cerebrovascular disorder, head trauma, multiple organ failure or extensive burn requiring intensive treatment), treatment or prophylaxis of ulcer caused by non-steroidal anti-inflammatory agent; treatment or prophylaxis of hyperacidity and ulcer due to postoperative stress and the like. For eradication or aid of eradication of Helicobacter pylori, a combined use of the solid preparation of the present invention and a penicillin antibiotic (e.g., amoxicillin and the like) and an erythromycin antibiotic (e.g., clarithromycin and the like) is preferable.
The solid preparation of the present invention is particularly preferably applied as an agent for the treatment or prophylaxis of GERD (Symptomatic GERD, erosive esophagitis and the like).
The solid preparation of the present invention can be directly administered orally. In addition, it is possible to disperse or dissolve the preparation in water, juice, yogurt and the like in advance, and administer the dispersion or solution in the form of a liquid or a semisolid.
The daily dose of the solid preparation of the present invention varies depending on the severity of symptom, the age, sex and body weight of the subject of administration, the timing and interval of administration, the kind of the active ingredient and the like, and is not particularly limited. For example, for oral administration, the dose of the active ingredient of a therapeutic drug for erosive esophagitis (GERD) is about 10 - 200 mg/day, preferably about 30 - 120 mg/day, for an adult (60 kg). The solid preparation of the present invention may be administered once a day or in 2 or 3 portions a day.

The solid preparation of the present invention obtained as mentioned above preferably shows an increase in the intragastric average pH to not less than 4 in about 0.5 hr after administration to a mammal, and the time of retention at pH 4 or above for one day of not less than 14 hr.

The absorption of the compound unstable to acid in the solid preparation of the present invention from the gastrointestinal tract is controlled by two kinds of systems utilizing the immediate release property of the compound unstable to acid in the immediate-release part and the sustained release property (prolongation of dwelling in the gastrointestinal tract) of the compound unstable to acid in the sustained-release part. When the solid preparation of the present invention is orally administered, the compound unstable to acid is released from the immediate-release part in the stomach immediately after administration, and rapidly exhibits a pharmacological effect. At this time, since an antacid is released in the stomach prior to the release of the compound unstable to acid, decomposition of the compound unstable to acid by gastric acid is suppressed, and the pharmacological effect can be exhibited rapidly and stably. In the meantime, the compound unstable to acid in the sustained-release part is gradually released from the pH-independent material as it moves in the gastrointestinal tract, and sequentially absorbed by respective gastrointestinal tracts (stomach, small intestine, large intestine and the like). As a result, the solid preparation of the present invention can afford both a rapid pharmacological effect after administration and a pharmacological effect sustained for a prolonged period of time. In addition, since the solid preparation of the present invention contains a basic substance in the immediate-release part and, when desired, the sustained-release part, the preparation is superior in the stability during the production and preservation.
Accordingly, the solid preparation of the present invention is useful as various preparations for oral administration.

### [Examples]

The present invention is explained in more detail in the following by referring to Preparation Examples, Examples and Experimental Examples, which are not to be construed as limitative.

### Preparation Example 1

### Production of sustained-release part (inner-core matrix tablet)

Lansoprazole (hereinafter to be sometimes referred to as compound A; 6.0 g), hydroxypropylmethylcellulose (trade name: Metolose 90SH-100SR, manufactured by Shin-Etsu Chemical Co., Ltd., 6.67 g), D-mannitol (5.07 g), crystalline cellulose (trade name: Ceolus PH-101, manufactured by Asahi Kasei Chemicals, 4.59 g), magnesium stearate (0.23 g) and Aerosil (1.1 g) were mixed in a mortar. The obtained mixture (170 mg) was tabletted (tabletting pressure: 1 ton/cm²) using an oil hydraulic pump pressing machine (manufactured by Riken Seiki) to give an inner-core matrix tablet having a diameter of 7 mm. This was used as the sustained-release part of the solid preparation of the present invention.

### Preparation Example 2

### Production of immediate-release part granulated powder

Compound A (10 g), calcium carbonate (166.67 g) and D-mannitol (155.8 g) were charged in a fluid bed granulator, the mixture was granulated while spraying an aqueous solution of hydroxypropylcellulose (13.87 g) in purified water (231.11 g), and the granules were dried to give a granulated powder (340 g) for the immediate-release part.

### Preparation Example 3

### Production of antacid-containing granulated powder

Magnesium hydroxide (96.67 g), magnesium oxide (133.33 g), D-mannitol (121.87 g) and crospovidone (10.68 g) were charged in a fluid bed granulator, the mixture was granulated while spraying an aqueous solution of hydroxypropylcellulose (13.42 g) in purified water (223.67 g), and the granules were dried to give a granulated powder (370 g) containing an antacid.

### Preparation Example 4

### Production of sustained-release part (inner-core matrix tablet)

Compound A (6.0 g), HPMC (trade name: Metolose 90SH-400SR, manufactured by Shin-Etsu Chemical Co., Ltd., 6.67 g), D-mannitol (5.07 g), crystalline cellulose (trade name: Ceolus PH-101, manufactured by Asahi Kasei Chemicals, 4.59 g), magnesium stearate (0.23 g) and Aerosil (1.1 g) were mixed in a mortar. The obtained mixture (170 mg) was tabletted (tabletting pressure: 1 ton/cm²) using an oil hydraulic pump pressing machine (manufactured by Riken Seiki) to give an inner-core matrix tablet having a diameter of 7 mm. This was used as the sustained-release part of the solid preparation of the present invention.

### Preparation Example 5

### Production of sustained-release part (inner-core matrix tablet)

Compound A (6.0 g), HPMC (trade name: Metolose 90SH-4000SR, manufactured by Shin-Etsu Chemical Co., Ltd., 6.67 g), D-mannitol (5.07 g), crystalline cellulose (trade name: Ceolus PH-101, manufactured by Asahi Kasei Chemicals, 4.59 g), magnesium stearate (0.23 g) and Aerosil (1.1 g) were mixed in a mortar. The obtained mixture (170 mg) was tabletted (tabletting pressure: 1 ton/cm²) using an oil hydraulic pump pressing machine (manufactured by Riken Seiki) to give an inner-core matrix tablet having a diameter of 7 mm. This was used as the sustained-release part of the solid preparation of the present invention.

### Preparation Example 6

### Production of sustained-release part (inner-core matrix tablet)

Compound A (22.5 g), POLYOX WSR 303 (trade name, manufactured by Union Carbide Corporation, 20.0 g) and PEG6000 (57.5 g) were mixed in a mortar. The obtained mixture (200 mg) was tabletted (tabletting pressure: 1 ton/cm²) using an oil hydraulic pump pressing machine (manufactured by Riken Seiki) to give an inner-core matrix tablet having a diameter of 7 mm. This was used as the sustained-release part of the solid preparation of the present invention.

### Preparation Example 7

### Production of sustained-release part (inner-core matrix tablet)

Compound A (22.5 g), POLYOX WSR 303 (trade name, manufactured by Union Carbide Corporation, 30.0 g) and PEG6000 (47.5 g) were mixed in a mortar. The obtained mixture (200 mg) was tabletted (tabletting pressure: 1 ton/cm²) using an oil hydraulic pump pressing machine (manufactured by Riken Seiki) to give an inner-core matrix tablet having a diameter of 7 mm. This was used as the sustained-release part of the solid preparation of the present invention.

### Preparation Example 8

### Production of sustained-release part (inner-core matrix tablet)

Compound A (22.5 g), POLYOX WSR 303 (trade name, manufactured by Union Carbide Corporation, 40.0 g) and PEG6000 (37.5 g) were mixed in a mortar. The obtained mixture (200 mg) was tabletted (tabletting pressure: 1 ton/cm²) using an oil hydraulic pump pressing machine (manufactured by Riken Seiki) to give an inner-core matrix tablet having a diameter of 7 mm. This was used as the sustained-release part of the solid preparation of the present invention.

### Preparation Example 9

### Preparation of core particle

Core particles to be the core of the sustained-release part were prepared as follows. Hydroxypropylcellulose (HPC-SL, 50 g) was dissolved in purified water (640 g), and low-substituted hydroxypropylcellulose (L-HPC-32W, 25 g) and magnesium carbonate (50 g) were added to the solution and dispersed therein. Compound A (150 g) was uniformly dispersed in the obtained dispersion to give a coating solution. Lactose·crystalline cellulose particles (Nonpareil 105, 100 g) were coated with this coating solution containing compound A (610 g) using a rotating fluidized bed coater(SPIR-A-FLOW, manufactured by Freund Industry Co., Ltd.). The coating conditions were inlet air temperature: about 60°C, spray air pressure: about 1 kgf/cm², exhaust air gauge: 100, BED pressure: about 250 mmHg, rotation speed of rotor: about 300 rpm, spray rate: about 6 g/min, and spray gun position: lower side. After the completion of coating operation, the obtained granule was vacuum-dried at 40°C for 16 hr, and sieved using a round sieve to give a core particle having 125 µm - 500 µm of particle size.

**[Table 1]**

| [Composition in core particle 85 mg] | |
|---|---|
| lactose·crystalline cellulose particle (Nonpareil 105) | 30 mg |
| compound A | 30 mg |
| magnesium carbonate | 10 mg |
| low-substituted hydroxypropylcellulose | 5 mg |
| hydroxypropylcellulose | 10 mg |
| total | 85 mg |

### Preparation Example 10

### Preparation of sustained-release part fine granules

The fine granules for the sustained-release part were prepared as follows. Polysorbate 80 (0.45 g) and triethyl citrate (4.5 g) were dissolved in purified water (109.4 g), and glycerol monostearate (1.13 g) was added and dispersed therein while heating at 70°C. The dispersion was allowed to cool to room temperature, and mixed with Eudragit RL30D (75 g) to give a coating solution. The core particles (100 g) obtained in Preparation Example 9 were coated with this coating solution (127 g) using a rotating fluidized bed coater (SPIR-A-FLOW, manufactured by Freund Industry Co., Ltd.). The coating conditions were inlet air temperature: about 35°C, spray air pressure: about 1 kgf/cm², exhaust air gauge: 100, BED pressure: about 250 mmHg, rotation speed of rotor: about 300 rpm, spray rate: about 2.7 g/min, and spray gun position: lower side. After the completion of coating operation, the obtained fine granules were vacuum-dried at 40°C for 24 hr, and sieved using a round sieve to give sustained-release part fine granules having 125 µm - 500 µm of particle size.

**[Table 2]**

| [Composition in sustained-release part fine granules 101.2 mg] | |
|---|---|
| core particle obtained in Preparation | 85 mg |
| Example 9 | |
| Eudragit RL30D | 12.75 mg |
| polysorbate 80 | 0.26 mg |
| glycerol monostearate | 0.64 mg |
| triethyl citrate | 2.55 mg |
| total | 101.2 mg |

### Preparation Example 11

### Preparation of sustained-release part fine granules

The fine granules for the sustained-release part were prepared as follows. Talc (22.5 g) was dissolved in purified water (127.5 g) and dispersed therein, and the obtained dispersion was mixed with Eudragit NE30D (75 g) to give a coating solution. The core particles (100 g) obtained in Preparation Example 9 were coated with this coating solution (50 g) using a rotating fluidized bed coater (SPIR-A-FLOW, manufactured by Freund Industry Co., Ltd.). The coating conditions were inlet air temperature: about 30°C, spray air pressure: about 1 kgf/cm², exhaust air gauge: 100, BED pressure: about 250 mmHg, rotation speed of rotor: about 300 rpm, spray rate: about 2.3 g/min and spray gun position: lower side. After the completion of coating operation, the obtained fine granules were vacuum-dried at 40°C for 24 hr, and sieved using a round sieve to give sustained-release part fine granules having 125 µm - 500 µm of particle size.

**[Table 3]**

| [Composition in sustained-release part fine granules 93.5 mg] | |
|---|---|
| core particle obtained in Preparation | 85 mg |
| Example 9 | |
| Eudragit NE30D | 4.25 mg |
| talc | 4.25 mg |
| total | 93.5 mg |

### Preparation Example 12

### Preparation of core particle

Core particles to be the core of the sustained-release part were prepared as follows. Hhydroxypropylmethylcellulose (TC-5EW, 50 g) was dissolved in purified water (640 g), and low-substituted hydroxypropylcellulose (L-HPC-32W, 25 g) and magnesium carbonate (50 g) were added to the solution and dispersed therein. Compound A (150 g) was uniformly dispersed into the obtained dispersion to give a coating solution. Lactose·crystalline cellulose particles (Nonpareil 105T, 130 g) were coated with this compound A-containing coating solution (793 g) using a rotating fluidized bed coater (SPIR-A-FLOW, manufactured by Freund Industry Co., Ltd.). The coating conditions were inlet air temperature: about 40°C, spray air pressure: about 1 kgf/cm², exhaust air gauge: 100, BED pressure: about 250 mmHg, rotation speed of rotor: about 300 rpm, spray rate: about 6 g/min and spray gun position: lower side. After the completion of coating operation, the obtained fine granules were vacuum-dried at 40°C for 16 hr, and sieved using a round sieve to give a core particle having 125 µm - 500 µm of particle size.

**[Table 4]**

| [Composition in core particle 85 mg] | |
|---|---|
| lactose·crystalline cellulose particle (Nonpareil 105T) | 30 mg |
| Compound A | 30 mg |
| magnesium carbonate | 10 mg |
| low-substituted hydroxypropylcellulose | 5 mg |
| hydroxypropylmethylcellulose | 10 mg |
| total | 85 mg |

### Preparation Example 13

### Preparation of sustained-release part fine granules

The fine granules for the sustained-release part were prepared as follows. Polysorbate 80 (0.45 g) and triethyl citrate (4.5 g) were dissolved in purified water (109.4 g), and glycerol monostearate (1.13 g) was added and dispersed therein while heating at 70°C. The dispersion was allowed to cool to room temperature, and mixed with Eudragit RS30D (75 g) to give a coating solution. Core particles (100 g) obtained in Preparation Example 12 were coated with this coating solution (127 g) using a rotating fluidized bed coater (SPIR-A-FLOW, manufactured by Freund Industry Co., Ltd.). The coating conditions were inlet air temperature: about 35°C, spray air pressure: about 1 kgf/cm², exhaust air gauge: 100, BED pressure: about 250 mmHg, rotation speed of rotor: about 300 rpm, spray rate: about 2.7 g/min and spray gun position: lower side. After the completion of coating operation, the obtained fine granules were vacuum-dried at 40°C for 24 hr, and sieved using a round sieve to give sustained-release part fine granules having 125 µm - 500 µm of particle size.

**[Table 5]**

| [Composition in sustained-release part fine granules 101.2 mg] | |
|---|---|
| core particle obtained in Preparation | 85 mg |
| Example 12 | |
| Eudragit RS30D | 12.75 mg |
| Polysorbate 80 | 0.26 mg |
| glycerol monostearate | 0.64 mg |
| triethyl citrate | 2.55 mg |
| total | 101.2 mg |

### Preparation Example 14

### Preparation of core particle

Core particles to be the core of the sustained-release part were prepared as follows. Hydroxypropylmethylcellulose (TC-5EW, 36 g) was dissolved in purified water (460.8 g), and low-substituted hydroxypropylcellulose (L-HPC-32W, 18 g) and magnesium carbonate (36 g) were added to the solution and dispersed therein. Compound A (108 g) was uniformly dispersed into the obtained dispersion to give a coating solution. Crystalline cellulose particles (Celphere SCP-100, 165 g) were coated with this compound A-containing coating solution (549 g) using a rotating fluidized bed coater (SPIR-A-FLOW, manufactured by Freund Industry Co., Ltd.). The coating conditions were inlet air temperature: about 45°C, spray air pressure: about 1 kgf/cm², exhaust air gauge: 40, BED pressure: about 120 mmHg, rotation speed of rotor: about 150 rpm, spray rate: about 5 g/min and spray gun position: lower side. After the completion of coating operation, the obtained fine granules were vacuum-dried at 40°C for 16 hr, and sieved using a round sieve to give core particles of having 125 µm - 500 µm of particle size.

**[Table 6]**

| [Composition in core particle 330 mg] | |
|---|---|
| crystalline cellulose particle (Celphere SCP-100) | 165 mg |
| compound A | 90 mg |
| magnesium carbonate | 30 mg |
| low-substituted hydroxypropylcellulose | 15 mg |
| hydroxypropylmethylcellulose | 30 mg |
| total | 330 mg |

### Preparation Example 15

### Preparation of sustained-release part fine granules

The fine granules for the sustained-release part were prepared as follows. Triethyl citrate (4.5 g) was dissolved in purified water (105 g) and talc (11.3 g) was added and dispersed therein. The dispersion was mixed with Eudragit RL30D (15 g) and Eudragit RS30D (60 g) to give a coating solution. Core particles (100 g) obtained in Preparation Example 14 were coated with this coating solution (131 g) using a rotating fluidized bed coater (SPIR-A-FLOW, manufactured by Freund Industry Co., Ltd.). The coating conditions were inlet air temperature: about 35°C, spray air pressure: about 1 kgf/cm², exhaust air gauge: 40, BED pressure: about 120 mmHg, rotation speed of rotor: about 150 rpm, spray rate: about 2.9 g/min and spray gun position: lower side. After the completion of coating operation, the obtained fine granules were vacuum-dried at 40°C for 24 hr, and sieved using a round sieve to give sustained-release part fine granules having 125 µm - 500 µm of particle size.

**[Table 7]**

| [Composition in sustained-release part fine granules 414.2 mg] | |
|---|---|
| core particles obtained in Preparation | 330 mg |
| Example 14 | |
| Eudragit RL | 9.9 mg |
| Eudragit RS | 39.6 mg |
| talc | 24.8 mg |
| triethyl citrate | 9.9 mg |
| total | 414.2 mg |

### Preparation Example 16

### Preparation of sustained-release part fine granules

The fine granules for the sustained-release part were prepared as follows. Triethyl citrate (4.5 g) was dissolved in purified water (105 g), and talc (11.3 g) was added and dispersed therein. The dispersion was mixed with Eudragit RS30D (75 g) to give a coating solution. Core particles (100 g) obtained in Preparation Example 14 were coated with this coating solution (87 g) using a rotating fluidized bed coater (SPIR-A-FLOW, manufactured by Freund Industry Co., Ltd.). The coating conditions were inlet air temperature: about 35°C, spray air pressure: about 1 kgf/cm², exhaust air gauge: 40, BED pressure: about 120 mmHg, rotation speed of rotor: about 150 rpm, spray rate: about 2.7 g/min and spray gun position: lower side. After the completion of coating operation, the obtained fine granules were vacuum-dried at 40°C for 24 hr, and sieved using a round sieve to give sustained-release part fine granules having 125 µm - 500 µm of particle size.

**[Table 8]**

| [Composition in sustained-release part fine granules 386.1 mg] | |
|---|---|
| core particles obtained in Preparation | 330 mg |
| Example 14 | |
| Eudragit RS30D | 33 mg |
| talc | 16.5 mg |
| triethyl citrate | 6.6 mg |
| total | 386.1 mg |

### Example 1

### Production of solid preparation (tablet)

The granulated powder (51.95 g) for the immediate-release part obtained in the above-mentioned Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder was weighed (530 mg), charged in a 14 mmφ flat-faced die with curved edge, and compressed gently. The inner-core matrix tablet prepared in Preparation Example 1 was placed at the center, and pressed gently with tweezers until about half of the inner-core matrix was buried therein. The above-mentioned mixed powder was weighed (530 mg) again and poured thereon, from which the solid preparation of the present invention (tablet, 1230 mg) containing compound A (60 mg) was prepared using Autograph (trade name, manufactured by Shimazu Co. Ltd., tabletting pressure: 1 ton/cm²). The obtained tablet showed no darkening.

### Example 2

### Production of solid preparation (tablet)

The granulated powder (51.95 g) for the immediate-release part obtained in the above-mentioned Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder was weighed (530 mg), charged in a 14 mmφ flat-faced die with curved edge, and compressed gently. The inner-core matrix tablet prepared in Preparation Example 4 was placed at the center, and pressed gently with tweezers until about half of the inner-core matrix was buried therein. The above-mentioned mixed powder was weighed (530 mg) again and poured thereon, from which the solid preparation of the present invention (tablet, 1230 mg) containing compound A (60 mg) was prepared using Autograph (trade name, manufactured by Shimazu Co. Ltd., tabletting pressure: 1 ton/cm²). The obtained tablet showed no darkening.

### Example 3

### Production of solid preparation (tablet)

The granulated powder (51.95 g) for the immediate-release part obtained in the above-mentioned Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder was weighed (530 mg), charged in a 14 mmφ flat-faced die with curved edge, and compressed gently. The inner-core matrix tablet prepared in Preparation Example 5 was placed at the center, and pressed gently with tweezers until about half of the inner-core matrix was buried therein. The above-mentioned mixed powder was weighed (530 mg) again and poured thereon, from which the solid preparation of the present invention (tablet, 1230 mg) containing compound A (60 mg) was prepared using Autograph (trade name, manufactured by Shimazu Co. Ltd., tabletting pressure: 1 ton/cm²). The obtained tablet showed no darkening.

### Example 4

### Production of solid preparation (tablet)

The granulated powder (51.95 g) for the immediate-release part obtained in the above-mentioned Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder was weighed (530 mg), charged in a 14 mmφ flat-faced die with curved edge, and compressed gently. The inner-core matrix tablet prepared in Preparation Example 6 was placed at the center, and pressed gently with tweezers until about half of the inner-core matrix was buried therein. The above-mentioned mixed powder was weighed (530 mg) again and poured thereon, from which the solid preparation of the present invention (tablet, 1260 mg) containing compound A (60 mg) was prepared using Autograph (trade name, manufactured by Shimazu Co. Ltd., tabletting pressure: 1 ton/cm²). The obtained tablet showed no darkening.

### Example 5

### Production of solid preparation (tablet)

The immediate-release part granulated powder (51.95 g) obtained in the above-mentioned Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder was weighed (530 mg), charged in a 14 mmφ flat-faced with curved edge, and compressed gently. The inner-core matrix tablet prepared in Preparation Example 7 was placed at the center, and pressed gently with tweezers until about half of the inner-core matrix was buried therein. The above-mentioned mixed powder was weighed (530 mg) again and poured thereon, from which the solid preparation of the present invention (tablet, 1260 mg) containing compound A (60 mg) was prepared using Autograph (trade name, manufactured by Shimazu Co. Ltd., tabletting pressure: 1 ton/cm²). The obtained tablet showed no darkening.

### Example 6

### Production of solid preparation (tablet)

The granulated powder (51.95 g) for the immediate-release part obtained in the above-mentioned Preparation Example 2, the antacid-containing granulated powder (37.60g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder was weighed (530 mg), charged in a 14 mmφ flat-faced die with curved edge, and compressed gently. The inner-core matrix tablet prepared in Preparation Example 8 was placed at the center, and pressed gently with tweezers until about half of the inner-core matrix was buried therein. The above-mentioned mixed powder was weighed (530 mg) again and poured thereon, from which the solid preparation of the present invention (tablet, 1260 mg) containing compound A (60 mg) was prepared using Autograph (trade name, manufactured by Shimazu Co. Ltd., tabletting pressure: 1 ton/cm²). The obtained tablet showed no darkening.

### Example 7

### Preparation of solid preparation (tablet)

The granulated powder (51.95 g) for the immediate-release part obtained in Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder (10.6 g) and the fine granule (2.06 g) for the sustained-release part prepared in Preparation Example 15 were mixed in a mortar to give a mixed powder. The obtained mixed powder (1266 mg) was charged in a 14 mmφ flat-faced die with curved edge, from which the solid preparation of the present invention (tablet, 1266 mg) containing compound A (60 mg) was prepared using Autograph (trade name, manufactured by Shimazu Co. Ltd., tabletting pressure: 1 ton/cm²). The obtained tablet showed no darkening.

### Example 8

### Preparation of solid preparation (tablet)

The granulated powder (51.95 g) for the immediate-release part obtained in Preparation Example 2, the antacid-containing granulated powder (37.60 g) obtained in the above-mentioned Preparation Example 3, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder (10.6 g) and the fine granules (1.93 g) for the sustained-release part prepared in Preparation Example 16 were mixed in a mortar to give a mixed powder. The obtained mixed powder (1253 mg) was charged in a 14 mmφ flat-faced die with curved edge, from which the solid preparation of the present invention (tablet, 1253 mg) containing compound A (60 mg) was prepared using Autograph (trade name, manufactured by Shimazu Co. Ltd., tabletting pressure: 1 ton/cm²). The obtained tablet showed no darkening.

### Industrial Applicability

According to the present invention, a solid preparation showing high stability of the active ingredient (compound unstable to acid), which expresses a pharmacological effect of the active ingredient stably and rapidly after administration, and sustains the pharmacological effect for a prolonged period of time can be provided. Accordingly, the solid preparation of the present invention is useful as various preparations for oral administration. Particularly, when the compound unstable to acid is a PPI, the solid preparation of the present invention can be useful for the treatment or prophylaxis of peptic ulcer (e.g., gastric ulcer, duodenal ulcer, anastomotic ulcer, Zollinger-Ellison syndrome and the like), gastritis, GERD (Gastroesophageal Reflux Diseases; erosive esophagitis, esophageal reflux unaccompanied by esophagitis (Symptomatic GERD) and the like), NUD (Non Ulcer Dyspepsia), gastric cancer (including gastric cancer associated with promotion of interleukin-1β production by genetic polymorphism of interleukin-1), stomach MALT lymphoma and the like, eradication or as an aid for eradication of Helicobacter pylori, suppression of peptic ulcer, acute stress ulcer and hemorrhagic stomach, upper gastrointestinal hemorrhage due to invasive stress (stress caused by major surgery requiring postoperative intensive management or cerebrovascular disorder, head trauma, multiple organ failure or extensive burn requiring intensive treatment), treatment or prophylaxis of ulcer caused by non-steroidal anti-inflammatory agent; treatment or prophylaxis of hyperacidity, ulcer and the like due to postoperative stress and the like.

This application is based on application No. 2005-378242 filed in Japan, the contents of which are incorporated hereinto by reference. The patent references and non-patent references cited herein are hereby incorporated in full by reference, to the extent that they have been disclosed herein.

## Claims

1. A controlled release solid preparation comprising (1) an antacid, (2) an immediate-release part comprising a compound unstable to acid and a basic substance, and (3) a sustained-release part containing a compound unstable to acid and a pH-independent material in combination.

2. The preparation of claim 1, further comprising a basic substance in the sustained-release part.

3. The preparation of claim 1, wherein the pH-independent material is a hydrophilic polymer.

4. The preparation of claim 3, wherein the hydrophilic polymer is one kind or a mixture of two or more kinds selected from the group consisting of hydroxypropylcellulose, hydroxypropylmethylcellulose, methylcellulose, polyethylene oxide, sodium carboxymethylcellulose, ethyl acrylate-methyl methacrylate-trimethylammonioethyl methacrylate chloride copolymer, methyl methacrylate-ethyl acrylate copolymer and vinyl acetate-polyvinylpyrrolidone polymer matrix.

5. The preparation of claim 3, wherein the hydrophilic polymer is hydroxypropylmethylcellulose.

6. The preparation of claim 3, wherein the hydrophilic polymer is polyethylene oxide.

7. The preparation of claim 3, wherein the sustained-release part has a hydrophilic polymer content of about 5 wt% - about 95 wt%.

8. The preparation of claim 1, wherein the sustained-release part is a tablet, granule or fine granule having a pH-independent diffusion-controlling film.

9. The preparation of claim 8, wherein the pH-independent diffusion-controlling film contains one kind or a mixture of two or more kinds selected from the group consisting of an ethyl acrylate-methyl methacrylate-trimethylammonioethyl methacrylate chloride copolymer, a methyl methacrylate-ethyl acrylate copolymer and ethylcellulose.

10. The preparation of claim 1, wherein the compound unstable to acid is a proton pump inhibitor (PPI).

11. The preparation of claim 10, wherein the PPI is a compound represented by the formula (I): 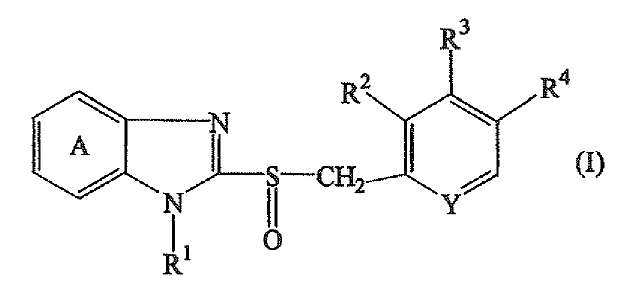 wherein ring A is a benzene ring optionally having substituent(s), R¹ is a hydrogen atom, an aralkyl group optionally having substituent(s), an acyl group or an acyloxy group, R², R³ and R⁴ are the same or different and each is a hydrogen atom, an alkyl group optionally having substituent(s), an alkoxy group optionally having substituent(s) or an amino group optionally having substituent(s), and Y is a nitrogen atom or CH, or an optically active form thereof or a salt thereof.

12. The preparation of claim 10, wherein the PPI is lansoprazole, omeprazole, rabeprazole, pantoprazole, ilaprazole or an optically active form thereof or a salt thereof.

13. The preparation of claim 1, wherein the antacid is at least one kind of component selected from the group consisting of a metal oxide, a metal hydroxide and an alkaline earth metal carbonate.

14. The preparation of claim 1, wherein a 1% aqueous solution or 1% aqueous suspension of the antacid has a pH of not less than 8.0.

15. The preparation of claim 13, wherein the metal oxide is at least one kind selected from the group consisting of magnesium oxide, magnesium silicate, dry aluminum hydroxide gel and magnesium aluminometasilicate.

16. The preparation of claim 13, wherein the metal hydroxide is at least one kind selected from the group consisting of magnesium hydroxide, aluminum hydroxide, synthetic hydrotalcite, coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate.

17. The preparation of claim 13, wherein the alkaline earth metal carbonate is calcium carbonate or magnesium carbonate.

18. The preparation of claim 1, wherein the content of the antacid is 5 mEq - 50 mEq.

19. The preparation of claim 1, wherein the weight ratio of the contents of the compound unstable to acid in the immediate-release part and the sustained-release part is 10:1 - 1:10.

20. The preparation of claim 1, which shows an increase in the intragastric average pH to 4 or above in 0.5 hr after oral administration to a mammal and a retention time at pH 4 or above of not less than 14 hr a day.

21. A solid preparation showing an increase in the intragastric average pH to 4 or above in 0.5 hr after oral administration to a mammal and a retention time at pH 4 or above of not less than 14 hr a day.
